# EUROPEAN PATENT APPLICATION

(11) **EP 4 437 855 A1**
(43) Date of publication of application: **02.10.2024**
(21) Application number: 22898622.0
(22) Date of filing: 24.11.2022
(51) Int. Cl.: A23L 5/00, A23C 11/10, A23L 2/38, A23L 11/60, A23L 11/65, C12N 9/04

(54) **PRODUCTION METHOD FOR VEGETABLE BEVERAGE AND FOOD, AND ENZYMATIC AGENT FOR LESSENING SUGARS**

(30) Priority: 25.11.2021 JP 2021191115
(71) Applicant: Amano Enzyme Inc., Nagoya-shi Aichi 460-8630 (JP)
(72) Inventor: TAKAYAMA Hiroyuki, Kakamigahara-shi, Gifu 509-0109 (JP)
(74) Representative: Forrest, Stuart
(86) International application number: PCT/JP2022/043400
(87) International publication number: WO 2023/095840

(57) **Abstract**

To provide a technique for reducing saccharides in plant-base drinks or foods.

The present technique provides a method for producing plant-base drinks or foods and a method for reducing saccharides in plant-base drinks or foods, which include a step for causing a maltotriose-generating enzyme to act on a plant-base raw material. In the production method according to the present technique, a step for causing a carbohydrate oxidase to act on a plant-base raw material can be further performed. The present technique also provides an enzymatic agent that is for reducing saccharides and that contains a maltotriose-generating enzyme. The enzymatic agent for reducing saccharides according to the present technique can further contain a carbohydrate oxidase.

## Description

### Technical Field

The present invention relates to a method for producing plant-base drinks or foods. More specifically, the present invention relates to a technique for reducing saccharides in plant-base drinks or foods using a maltotriose-generating enzyme.

### Background Art

In recent years, plant-base drinks or foods are attracting attention because of increasing health consciousness. For example, oat drinks are prepared by suspending an oat material in an aqueous medium and breaking down the starch of the oat material using one or more amylases. The amylase used at this process is β-amylase or a mixture of α-amylase and β-amylase. Oat drinks prepared by this method exhibit moderate sweetness due to the generation of maltose through the action of β-amylase (Patent Literature 1).

Here, the enzyme used in this technique will be described. Maltotriose-generating enzymes are enzymes that catalyze the reaction of hydrolyzing oligosaccharides contained in starch at maltotriose units. It is known that maltotriose-generating enzymes are used in the production of maltotriose syrup (Patent Literature 2).

A carbohydrate oxidase is an enzyme that has the property of oxidizing carbohydrates. A carbohydrate oxidase is known to be used in the production of saccharic acids typified by maltobionic acid (Patent Literature 3).

### Citation List

### Patent Literature

Patent Literature 1: Japanese Patent Application Publication No. 2018-075029
Patent Literature 2: Japanese Patent Application Publication No. Hei03-251173
Patent Literature 3: International Publication WO2014/042237

### Summary of Invention

### Technical Problem

As mentioned above, plant-base drinks or foods have attracted attention in recent years due to the increasing health consciousness, and for example, plant-base drinks or foods such as oat drinks or the like obtained by treating with β-amylase in addition to α-amylase using a technique shown in Patent Literature 1 described above contain a large amount of saccharides such as glucose and maltose. Therefore, the demand for reducing saccharide content has not been met.

Therefore, the main purpose of the present technique is to provide a technique for reducing saccharides in plant-base drinks or foods.

### Solution to Problem

The inventors of the present application have conducted intensive research on the technique for reducing saccharides in plant-base drinks or foods, and resultantly found that it was possible to reduce saccharides in the plant-base drinks or foods by causing a maltotriose-generating enzyme to act on the plant-base raw material of plant-base drinks or foods, leading to completion of the present technique.

That is, the present technique provides a method for producing plant-base foods or drinks, which includes a step of causing a maltotriose-generating enzyme to act on the plant-base raw material.

As the plant-base raw material used in the present technique, a raw material treated with α-amylase can be used.

In the production method according to the present technique, a step of causing a carbohydrate oxidase to act on the plant-base raw material can be further performed.

With the production method according to the present technique, oat milk can be produced as the plant-base food or drink.

The present technique also provides an enzymatic agent for reducing saccharides that includes a maltotriose-generating enzyme.

The enzymatic agent for reducing saccharides according to the present technique can further include a carbohydrate oxidase.

The present technique further provides a method for reducing saccharides in plant-base drinks or foods, which includes a step of causing a maltotriose-generating enzyme to act on a plant-base raw material.

In the present technique, "saccharide" is a general term for monosaccharides and disaccharides.

### Advantageous Effects of Invention

According to the present technique, saccharides in plant-base drinks or foods can be reduced.

### Description of Embodiments

Hereinafter, preferred embodiments for implementing the present technique will be described. Note that the embodiment described below shows an example of a typical embodiment of the present technique, and that the scope of the present technique should not be interpreted narrowly.

### <1. Method for producing plant-base drink or food, method for reducing saccharide in plant-base drink or food>

The method for producing plant-base drinks or foods and the method for reducing saccharides in plant-base drinks or foods according to the present technique are methods that at least implement a step of causing a maltotriose-generating enzyme to act on a plant-base raw material (hereinafter also referred to as "maltotriose-generating enzyme acting step"). Additionally, a step in which a carbohydrate oxidase is allowed to act on a plant-base raw material (hereinafter also referred to as "carbohydrate oxidase acting step") and a step in which α-amylase is allowed to act on a plant-base raw material (hereinafter also referred to as "α-amylase acting step") can also be performed. In addition, depending on the type of plant-base foods or drinks or the like, it is also possible to perform general food production steps before, after, or simultaneously with each step to the extent that the effects of the present technique are not impaired. In addition, in the method for producing plant-base drinks or foods and the method for reducing saccharides in plant-base drinks or foods according to the present technique, a recovery step of recovering the produced plant-base drinks or foods and the plant-base drinks or foods with reduced saccharide content can also be implemented. Each step will be described in detail below.

### (1) Plant-base raw material

The origin, type, etc. of the plant-base raw materials that can be used in this technique are not particularly limited, and can be freely selected depending on the desired plant-base food or drink, as long as the effects of this technique are not impaired. Examples thereof include legumes such as soybeans, green peas, lentils, chickpeas, black beans, fava beans, mung beans, lupin beans, kidney beans and the like; grains such as wheat, barley, oats, rice, rye, buckwheat, Japanese millet, foxtail millet, teff and the like; nuts such as almonds, coconuts, peanuts, cashews, hazelnuts, pecans, macadamia nuts, pistachios, walnuts, Brazil nuts, pili nuts, chestnuts, sesame seeds, pine nuts and the like; and hemp seeds (industrial hemp), chia seeds, quinoa, amaranthus, canary seeds, flaxseed and the like. In the present technique, these may be used alone or in combination of two or more. Among these raw materials, cereals are preferred, and oats are more preferred.

In the present technique, the properties of the plant-base raw materials when subjected to various enzyme treatments are not particularly limited as long as they do not impair the effects of the present technique, but preferably include liquid, slurry, and paste.

In this technique, when the plant-base raw material is treated with maltotriose-generating enzymes and/or carbohydrate oxidases described later, it is preferable to use α-amylase-treated plant-base raw materials. By treating plant-base raw materials with α-amylase, the viscosity of the raw materials can be reduced.

When using a plant-base raw material that has been treated with α-amylase, it is possible to use a plant-base raw material that has already been treated with α-amylase, or it is also possible to perform an α-amylase acting step described later.

### (2) Maltotriose-generating enzyme

The maltotriose-generating enzyme that can be used in the present technique is an enzyme that acts on starch and has an activity of mainly producing maltotriose. The maltotriose-generating enzyme that can be used in the present technique may be an enzyme that additionally has other functions as long as it has the activity of generating maltotriose.

With this technique, by causing a maltotriose-generating enzyme to act on a plant-base raw material, it is possible to reduce the amount of saccharides (monosaccharides and disaccharides) in produced plant-base drinks or foods. In addition, by causing a maltotriose-generating enzyme to act on a plant-base raw material, the amount of trisaccharides in the produced plant-base food or drink can be increased, thereby imparting appropriate sweetness.

The origin of the maltotriose-generating enzyme that can be used in the present technique is not particularly limited, and mentioned are maltotriose-generating enzymes derived from organisms of the genus Streptomyces, the genus Bacillus, the genus Microbacterium, and the genus Cellulosimicrobium, for example. These maltotriose-generating enzymes may be used alone or in combination of two or more. Among these maltotriose-generating enzymes, maltotriose-generating enzymes derived from organisms of the genus Microbacterium or organisms of the genus Cellulosimicrobium are preferably mentioned, and more preferably maltotriose-generating enzymes derived from Microbacterium sp. are mentioned. Specifically, enzymes that exhibit substrate specificity that acts on amylose, amylopectin, glycogen, and starch can be used.

"Microbacterium sp.-derived maltotriose-generating enzyme" herein refers to a maltotriose-generating enzyme produced by a microorganism (which may be a wild strain or a mutant strain) classified as Microbacterium sp., or a maltotriose-generating enzyme obtained by genetic engineering using a maltotriose-generating enzyme gene. Therefore, recombinants produced by host microorganisms into which a maltotriose-generating enzyme gene obtained from Microbacterium sp. (or a gene obtained by modifying the gene) is introduced also fall under "Microbacterium sp.-derived maltotriose-generating enzyme".

The maltotriose-generating enzyme used in the present technique can be prepared from a culture solution of a microorganism from which the above-mentioned maltotriose-generating enzyme is derived. A specific preparation method includes a method of recovering the maltotriose-generating enzyme from the culture solution or bacterial body of the above-mentioned microorganism. For example, when using a maltotriose-generating enzyme-secreting microorganism, the enzyme can be separated and/or purified after the bacterial bodies have been recovered from the culture solution by filtration, centrifugation, etc., if necessary. In addition, when using a maltotriose-generating enzyme non-secreting microorganism, if necessary, after recovering the bacterial bodies from the culture solution in advance, the bacterial bodies are crushed by pressure treatment, ultrasonication, etc. to expose the enzyme, and then, the enzyme can be separated and/or purified. As the enzyme separation and/or purification method, any known protein separation and/or purification method can be used without particular limitation, such as centrifugation method, UF concentration method, salting out method, various chromatography methods using ion exchange resins, etc. The separated and/or purified enzyme can be powdered by a drying method such as freeze drying or vacuum drying, and may be powdered using an appropriate excipient and/or drying aid in the drying method. Furthermore, the separated and/or purified enzyme can be liquefied by adding appropriate additives and sterilizing it by filtration.

In this technique, a commercially available product can also be used as the maltotriose-generating enzyme, and a preferred example of the commercially available product is AMT1.2L manufactured by Amano Enzyme Inc.

Various conditions for the maltotriose-generating enzyme acting step can be freely set as long as they do not impair the effects of the present technique. For example, the pH, temperature, action time, etc. can be set depending on the physicochemical properties such as the optimum pH, stable pH range, optimum temperature, and temperature stability of the maltotriose-generating enzyme used. The pH can be set, for example, to pH 5.0 to 8.0, preferably pH 5.5 to 7.5, more preferably pH 6.0 to 7.0. The temperature can be set to, for example, 30°C to 70°C, preferably 35°C to 65°C, more preferably 40°C to 60°C. The action time can be set, for example, from 10 minutes to 12 hours, preferably from 30 minutes to 6 hours, and more preferably from 1 hour to 3 hours. Note that the optimal reaction conditions can be determined through preliminary experiments.

The amount of maltotriose-generating enzyme added in the maltotriose-generating enzyme acting step can be freely set as long as it does not impair the effects of the present technique. The amount used per 1 g of a plant-base raw material is, for example, 0.1 U or more. From the viewpoint of further enhancing the saccharide reduction effect and the sweetness enhancement effect, the amount of maltotriose-generating enzyme used per 1 g of a plant-base raw material can be set to preferably 0.5 U or more, more preferably 1 U or more, even more preferably 5 U or more, further more preferably 10U or more. The upper limit of the amount of maltotriose-generating enzyme used per 1 g of a plant-base raw material is not particularly limited, but can be set to, for example, 1500 U or less, 300 U or less, 150 U or less, 100 U or less, 50 U or less, 20 U or less, or 15 U or less.

In addition, in this technique, the activity of the maltotriose-generating enzyme is a value measured by the starch saccharification power activity measurement method described in the examples below.

### (3) Carbohydrate oxidase

The carbohydrate oxidase that can be used in the present technique is not particularly limited as long as it is an enzyme that can oxidize carbohydrates, but enzymes that oxidize oligosaccharides of disaccharide or higher are preferred. Specifically, proteins having physicochemical properties described below can be mentioned.

With this technique, by causing a carbohydrate oxidase to act on a plant-base raw material, it is possible to further reduce the amount of saccharides (monosaccharides and disaccharides) in the produced plant-base drink or food.

### (A) Action

Carbohydrate oxidases that can be used in the present technique oxidize saccharides which will be described later to produce saccharic acids in the presence of oxygen. More specifically, when a carbohydrate oxidase that can be used in the present technique is allowed to act on a saccharide described below in the presence of oxygen, saccharic acid and hydrogen peroxide are generated.

### (B) Substrate specificity

As the carbohydrate oxidase that can be used in this technique, proteins that exhibit activity against one or more carbohydrates selected from glucose, maltotriose, maltose, galactose, maltotetraose, lactose, cellobiose, and dextrin can be used. The relative activity for each substrate is; maltotriose: about 92%, maltose: about 86%, galactose: about 79%, maltotetraose: about 60%, lactose: about 58%, cellobiose: about 53%, and maltodextrin: about 24%, when the activity for glucose is 100%.

In addition, in the present technique, if the relative activity is 50% or more when the activity when glucose is used as a substrate is the standard (100%), it is determined that "it is a substrate on which the present enzyme acts well".

In this way, by using a carbohydrate oxidase that is active not only on monosaccharides such as glucose but also on a wide range of carbohydrates including disaccharides and higher, it is possible to activate carbohydrate oxidation in a wide range of fields that cannot be addressed by existing glucose oxidases and oligosaccharide oxidases.

### (C) Km value

In this technique, the specific method for calculating the Km value (Michaelis constant) of a protein is not particularly limited, and any known method can be freely selected for calculation. Examples of methods for calculating the Km value of a protein include Lineweaver-Burk plot, Eadie-Hofstee plot, Hanes-Woolf plot and the like, and Hanes-Woolf plot is preferred. The Km value of the carbohydrate oxidase that can be used in the present technique is not particularly limited, but it is preferable that [Km value of glucose]/[Km value of maltose] <_ 1, and it is more preferable that 0.4 ≤ [Km value of glucose]/[Km value of maltose] <_ 1.

### (D) Molecular mass

As the carbohydrate oxidase that can be used in this technique, a carbohydrate oxidase having a molecular mass of about 63 kDa as determined by SDS-PAGE method can be used.

### (E) Optimal pH

As the carbohydrate oxidase that can be used in this technique, a carbohydrate oxidase that has the highest carbohydrate oxidase activity at around pH 5.0 to 9.0 under reaction conditions of 37°C for 5 minutes can be used.

### (F) Stable pH range

As the carbohydrate oxidase that can be used in this technique, a carbohydrate oxidase that is stable at around pH 5.0 to 10.5 under treatment conditions of 37°C for 15 minutes can be used.

### (G) Optimal temperature

As the carbohydrate oxidase that can be used in this technique, a carbohydrate oxidase that has the highest carbohydrate oxidase activity at around 20°C to 55°C under reaction conditions of pH 7.0 for 5 minutes can be used.

### (H) Temperature stability

As the carbohydrate oxidase that can be used in this technique, a carbohydrate oxidase that can maintain an activity of 80% or more even when treated under reaction conditions of pH 7.0 for 15 minutes and at temperatures up to 45°C can be used.

### (I) Regarding origin

The origin of the carbohydrate oxidase that can be used in the present technique described above is not particularly limited, but examples thereof include those derived from microorganisms belonging to the genus Acremonium. In this case, the microorganism belonging to the genus Acremonium includes Acremonium chrysogenum.

Here, "carbohydrate oxidase derived from Acremonium chrysogenum" refers to a carbohydrate oxidase produced by a microorganism (which may be a wild strain or a mutant strain) classified as Acremonium chrysogenum, or a carbohydrate oxidase obtained by genetic engineering using a carbohydrate oxidase gene. Therefore, recombinants produced by a host microorganism into which a carbohydrate oxidase gene obtained from Acremonium chrysogenum (or a gene obtained by modifying the gene) is introduced also fall under "carbohydrate oxidase derived from Acremonium chrysogenum".

Examples of Acremonium chrysogenum from which carbohydrate oxidases that can be used in the present technique are derived include Acremonium chrysogenum NBRC30055 (NITE, Japan), ATCC 15006 (ATCC, USA), and DSM880 (DSMZ, Germany).

### (J) Amino acid sequence

Although the amino acid structure of the carbohydrate oxidase that can be used in the present technique is not limited, one example can be specified by the following amino acid sequence.

Specifically, the carbohydrate oxidase that can be used in the present technique can be specified by the amino acid sequence represented by SEQ ID NO: 1.

Generally, when a part of the amino acid sequence of a certain protein is modified, the modified protein may have the same function as the unmodified protein. That is, modification of the amino acid sequence may not substantially affect the function of the protein, and the function of the protein may be maintained before and after the modification. Therefore, as another aspect of the present invention, the present invention provides a protein having carbohydrate oxidase activity, which includes an amino acid sequence in which one to several amino acids are deleted, substituted, and/or added in the amino acid sequence represented by SEQ ID NO: 1. "Deletion, substitution, and/or addition of one to several amino acids constituting an amino acid sequence" typically refers to a partial difference in the amino acid sequence.

The difference in the amino acid sequence here is permissible as long as carbohydrate oxidase activity can be maintained (some variation in activity may be allowed). As long as this condition is met, the positions where the amino acid sequences differ are not particularly limited, and differences may occur at a plurality of positions. The plurality here refers to, for example, a number corresponding to less than about 30% of the total amino acid sequence, preferably a number corresponding to less than about 20%, further preferably a number corresponding to less than about 10%, even more preferably a number representing less than about 5%, and most preferably a number representing less than about 1%.

That is, it refers to having, for example, identity of about 70% or more, preferably about 80% or more, more preferably about 90% or more, even more preferably about 95% or more, most preferably about 99% or more, with the amino acid sequence of SEQ ID NO: 1.

In addition, a method of obtaining a protein by generating conservative amino acid substitutions in amino acid residues that are not essential for carbohydrate oxidase activity is preferable. The term "conservative amino acid substitution" as used herein refers to replacing a certain amino acid residue with an amino acid residue having a side chain with similar properties. Amino acid residues are classified into several families such as basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), β-branched side chains (e.g., threonine, valine, isoleucine), and aromatic side chains (e.g., tyrosine, phenylalanine, tryptophan, histidine), according to their side chains. Conservative amino acid substitutions are preferably substitutions between amino acid residues within the same family.

Here, the identity (%) of two amino acid sequences or two nucleic acids (hereinafter, the term "two sequences" will be used to include these) can be determined, for example, by the following procedure. First, two sequences are aligned for optimal comparison. At this procedure, for example, a gap may be introduced into the first sequence to optimize alignment with the second sequence. When a molecule (amino acid residue or nucleotide) at a particular position in the first sequence is the same as a molecule at the corresponding position in the second sequence, the molecules at that position can be said to be identical. The identity of two sequences is a function of the number of identical positions common to the two sequences (i.e., identity (%) = number of identical positions/total number of positions × 100), and preferably, the number and size of gaps required for alignment optimization are also taken into account.

Comparison of two sequences and determination of identity can also be accomplished using mathematical algorithms. Specific examples of mathematical algorithms that can be used to compare sequences include, but are not limited to, an algorithm that is described in Karlin and Altschul (1990) Proc. Natl. Acad. Sci. USA 87: 2264-68 and modified in Karlin and Altschul (1993) Proc. Natl. Acad. Sci. USA 90: 5873-77. Such an algorithm is incorporated into NBLAST program and XBLAST program (version 2.0) described in Altschul et al. (1990) J. Mol. Biol. 215: 403-10. To obtain a nucleotide sequence equivalent to the nucleic acid molecule of the present invention, for example, a BLAST nucleotide search may be performed using the NBLAST program with score=100 and wordlength=12.

To obtain an amino acid sequence equivalent to the polypeptide molecule of the present invention, for example, a BLAST polypeptide search may be performed using the XBLAST program with score=50 and wordlength=3. To obtain gap alignments for comparison, Gapped BLAST described in Altschul et al. (1997) Amino Acids Research 25 (17): 3389-3402 can be used. When using BLAST and Gapped BLAST, the default parameters of the corresponding programs (e.g., XBLAST and NBLAST) can be used. For more information, please refer to http://www.ncbi.nlm.nih.gov.

Examples of other mathematical algorithms available for comparing sequences include an algorithm described in Myers and Miller (1998) Comput Appl Biosci. 4: 11-17. Such an algorithm is incorporated into the ALIGN program available for example on the GENESTREAM network server (IGH Montpellier, France) or on the ISREC server. When using the ALIGN program to compare amino acid sequences, for example, the PAM120 residue mass table can be used and the gap length penalty can be set to 12 and the gap penalty to be 4.

The carbohydrate oxidase that can be used in the present technique may be part of a larger protein (e.g., a fusion protein). Sequences added in the fusion protein include, for example, sequences useful for purification such as multiple histidine residues, additional sequences that ensure stability during recombinant production, and the like.

A protein having the above amino acid sequence can be easily prepared by genetic engineering techniques. For example, it can be prepared by transforming a suitable host cell (e.g., Escherichia coli, yeast, filamentous fungus) with DNA encoding the present protein and recovering the protein expressed in the transformant. The recovered protein is appropriately prepared depending on the purpose. If this protein is obtained as a recombinant protein in this way, various modifications are possible. For example, if the DNA encoding the present protein and other appropriate DNA are inserted into the same vector and the vector is used to produce a recombinant protein, this protein composed of a recombinant protein in which any peptide or protein is linked can be obtained. Further, addition of saccharide chains and/or lipids, or modifications that result in processing of the N-terminus or C-terminus may be performed. The above-mentioned modifications make it possible to simplify the extraction and purification of recombinant proteins, or to add biological functions, or the like.

Various conditions for the carbohydrate oxidase acting step can be freely set as long as they do not impair the effects of the present technique. For example, the pH, temperature, action time, etc. can be set depending on the physicochemical properties such as the optimum pH, stable pH range, optimum temperature, and temperature stability of the carbohydrate oxidase used. The pH can be set, for example, to pH 4.0 to 8.0, preferably pH 4.5 to 7.5, more preferably pH 5.0 to 7.0. The temperature can be set to, for example, 20°C to 60°C, preferably 25°C to 55°C, more preferably 30°C to 50°C. The action time can be set, for example, from 10 minutes to 12 hours, preferably from 30 minutes to 6 hours, and more preferably from 1 hour to 3 hours. Note that the optimal reaction conditions can be determined through preliminary experiments.

The amount of a carbohydrate oxidase added in the carbohydrate oxidase acting step can be freely set as long as the effect of the present technique is not impaired. The amount used per 1 g of a plant-base raw material is, for example, 0.1 U or more. From the viewpoint of further enhancing the effect, the amount of a carbohydrate oxidase used per 1 g of a plant-base raw material is preferably 1 U or more, more preferably 5 U or more, still more preferably 10 U or more, even more preferably 20 U or more. The upper limit of the use amount range of a carbohydrate oxidase per 1 g of a plant-base raw material is not particularly limited, but includes, for example, 300 U or less, 200 U or less, 100 U or less, preferably 50 U or less, more preferably 30 U or less, still more preferably 25 U or less.

In addition, in this technique, the activity of a carbohydrate oxidase is a value measured by the glucose oxidase activity measurement method described in examples described later.

The order in which the carbohydrate oxidase acting step is performed is not limited as long as it does not impair the effects of the present technique, but it is preferably performed after the maltotriose-generating enzyme acting step or simultaneously with the maltotriose-generating enzyme acting step. Further, an enzyme deactivation step may be performed after each enzyme acting step.

### (4) α-Amylase

The α-amylase that can be used in the present technique is an enzyme that acts on starch and mainly hydrolyzes α-1,4 glycosidic bond.

The origin of the α-amylase that can be used in the present technique is not particularly limited, but examples thereof include α-amylase derived from organisms of the genus Aspergillus and Bacillus, preferably those from organisms of the genus Bacillus, and more preferably α-amine derived from Bacillus subtilis, Bacillus amyloliquefaciens, Bacillus licheniformis, more preferably α-amine derived from Bacillus amyloliquefaciens.

Various conditions for the α-amylase acting step can be freely set as long as they do not impair the effects of the present technique. For example, the pH, temperature, action time, etc. can be set depending on the physicochemical properties of the α-amylase used, such as the optimum pH, stable pH range, optimum temperature, and temperature stability. The pH can be set, for example, to pH 5.0 to 8.0, preferably pH 5.5 to 7.5, more preferably pH 6.0 to 7.0. The temperature can be set to, for example, 30°C to 80°C, preferably 40°C to 75°C, more preferably 50°C to 70°C. The action time can be set, for example, from 10 minutes to 12 hours, preferably from 30 minutes to 6 hours, and more preferably from 1 hour to 3 hours. Note that the optimal reaction conditions can be determined through preliminary experiments.

The order in which the α-amylase acting step is performed is not limited as long as it does not impair the effects of the present technique, but it is preferably performed before the maltotriose-generating enzyme acting step or simultaneously with the maltotriose-generating enzyme acting step. Further, an enzyme deactivation step may be performed after each enzyme acting step.

### (5) Recovery step

The recovery step is a step of recovering the plant-base drink or food produced through a maltotriose-generating enzyme acting step, and if necessary, a carbohydrate oxidase acting step, an α-amylase acting step, and the like. As a specific recovery method, one type or a combination of two or more types of recovery methods commonly used in the production of plant-base drinks or foods can be used depending on the type of the plant-base drink or food to be produced.

The plant-base drink or food produced through a maltotriose-generating enzyme acting step and, if necessary, a carbohydrate oxidase acting step, an α-amylase acting step, etc. is characterized by having reduced saccharides (monosaccharides and disaccharides). Specifically, for example, the content of saccharides (monosaccharides and disaccharides) in the plant-base drink or food produced through a maltotriose-generating enzyme acting step and, if necessary, a carbohydrate oxidase acting step, an α-amylase acting step, etc. is 20 mg or less, preferably 15 mg or less, more preferably 10 mg or less per 1 mL or 1 g of the plant-base drink or food. That is, the present technique can implement a recovery step of recovering a plant-base drink or food with a content of saccharides (monosaccharides and disaccharides) of 20 mg or less, preferably 15 mg or less, and more preferably 10 mg or less per 1 mL or 1 g of the plant-base drink or food.

In addition, in the plant-base drink or food produced through a maltotriose-generating enzyme acting step and, if necessary, a carbohydrate oxidase acting step, an α-amylase acting step, etc., the amount of trisaccharides is increased, in addition to reduction of saccharides (monosaccharides and disaccharides). Specifically, for example, the content of trisaccharides in the plant-base drink or food produced through a maltotriose-generating enzyme acting step and, if necessary, a carbohydrate oxidase acting step, an α-amylase acting step, etc. is 8 mg or more, preferably 15 mg or more, more preferably 20 mg or more per 1 mL or 1 g of the plant-base drink or food. That is, in the present technique, it is possible to perform a recovery step of recovering a plant-base drink or food having a trisaccharide content of 8 mg or more, preferably 15 mg or more, and more preferably 20 mg or more per 1 mL or 1 g of the plant-base drink or food.

One aspect of the method for producing plant-base drinks or foods and the method for reducing saccharides in plant-base drinks or foods according to the present technique includes the following steps (1) and (2). In addition, (3) may also be included. Note that after the step (2) or (3), (4) a step of inactivating the enzyme, and/or (5) a step of recovering the plant-base drink or food with a reduction in saccharides (monosaccharides and disaccharides) and/or an increase in the amount of trisaccharides, may be added.
(1) A step of preparing a raw material containing a plant-base carbohydrate
(2) A step of treating the prepared raw material with a maltotriose-generating enzyme
(3) A step of treating the raw material treated with a maltotriose-generating enzyme with a carbohydrate oxidase

One aspect of the method for producing plant-base drinks or foods and the method for reducing saccharides in plant-base drinks or foods according to the present technique includes the following steps (1) to (3). In addition, (4) may also be included. Note that (5) a step of deactivating the enzyme may be added, and (6) a step of recovering the plant-base drink or food with a reduction in saccharides (monosaccharides and disaccharides) and/or an increase of the amount of trisaccharides may be added, after the step (2), (3) or (4).
(1) A step of preparing a raw material containing a plant-base carbohydrate
(2) A step of treating the prepared raw material with α-amylase
(3) A step of treating the raw material treated with α-amylase with a maltotriose-generating enzyme
(4) A step of treating with a carbohydrate oxidase

### <2. Enzyme agent for saccharide reduction>

The enzymatic agent for saccharide reduction according to the present technique contains a maltotriose-generating enzyme as an active ingredient. Furthermore, it is also possible to contain a carbohydrate oxidase and/or α-amylase, if necessary. The details of the maltotriose-generating enzyme, carbohydrate oxidase, and α-amylase are the same as the enzymes that can be used in the above-described method for producing plant-base drinks or foods, and therefore will not be described here.

The enzymatic agent for saccharide reduction may be composed only of the above-mentioned maltotriose-generating enzyme, as long as it contains the above-mentioned maltotriose-generating enzyme, or it is also possible to freely select and contain one or more types of other components as long as the effects of the present technique are not impaired. Other components that can be used include, for example, excipients, pH adjusters, coloring agents, flavoring agents, disintegrants, lubricants, stabilizers, and the like that are commonly used in formulations. Furthermore, components having functions that are known or that will be discovered in the future may be used in combination depending on the purpose.

### <3. Low-saccharide plant-base drink or food>

The low-saccharide plant-base drink or food according to the present technique is a plant-base drink or food produced using the above-described production method. Specific examples of the plant-base drink include oat drinks, and more specifically oat milk (also referred to as "oats milk"). A specific example of the plant-base food is plant-base yogurt.

The amount of saccharides (monosaccharides and disaccharides) contained in the low-saccharide plant-base drinks or foods according to the present technique is not particularly limited. For example, the amount of saccharides contained in the plant-base drink or food is 20 mg or less, preferably 15 mg or less, and more preferably 10 mg or less per 1 mL or 1 g of the plant-base drink or food.

The low-saccharide plant-base drinks or foods according to the present technique include those that have an increased amount of trisaccharides in addition to reduced amounts of saccharides (monosaccharides and disaccharides). Appropriate sweetness can be imparted by increasing trisaccharides. The amount of trisaccharides contained in the low-saccharide plant-base drink or food according to the present technique is also not particularly limited. For example, the amount of trisaccharides contained in the plant-base drink or food is 8 mg or more, preferably 15 mg or more, and more preferably 20 mg or more per 1 mL or 1 g of the plant-base food or drink.

Furthermore, the ratio of trisaccharides to saccharides (monosaccharides + disaccharides), which is (trisaccharides/saccharides (monosaccharides + disaccharides)), is not particularly limited, but in order to obtain both a saccharide reduction effect and a sweetness enhancement effect, it is, for example, 0.5 or more, preferably 0.8 or more, more preferably 1 or more, even more preferably 2 or more.

Note that the present technique can have the following configuration.
[1] A method for producing a plant-base drink or food, which comprises a step of causing a maltotriose-generating enzyme to act on a plant-base raw material.
[2] The method for producing a plant-base food or drink according to [1], wherein the plant-base raw material is a raw material treated with α-amylase.
[3] The method for producing a plant-base food or drink according to [1] or [2], further comprising a step of causing a carbohydrate oxidase to act on the plant-base raw material.
[4] The method for producing a plant-base drink or food according to any one of [1] to [3], wherein the plant-base drink or food is oat milk.
[5] An enzymatic agent for reducing saccharides, comprising a maltotriose-generating enzyme.
[6] The enzymatic agent for reducing saccharides according to [5], further comprising a carbohydrate oxidase.
[7] A method for reducing saccharides in plant-base drinks or foods, which comprises a step of causing a maltotriose-generating enzyme to act on a plant-base raw material.

### EXAMPLES

Hereinafter, the present invention will be described in more detail based on examples. It should be noted that the embodiment described below shows one example of a typical embodiment of the present invention, and the scope of the present invention should not be interpreted narrowly thereby.

1. Enzyme used
   (1) α-Amylase: "KLEISTASE SD8" (manufactured by Amano Enzyme Inc., α-amylase derived from Bacillus amyloliquefaciens)
   (2) β-Amylase: "β-Amylase F 'Amano'" (manufactured by Amano Enzyme Inc., β-amylase derived from Bacillus flexus)
   (3) Maltotriose-generating enzyme: "AMT1.2L" manufactured by Amano Enzyme Inc., maltotriose-generating enzyme derived from Microbacterium sp.)
   (4) Carbohydrate oxidase: In this example, a carbohydrate oxidase (derived from Acremonium chrysogenum) purified by the method described in WO2014/042237 was used as an example of the carbohydrate oxidase.
2. Enzyme activity measurement method

### [Method for measuring starch saccharification power activity: Method for measuring maltotriose-generating enzyme activity]

An enzyme was caused to act on soluble starch as a substrate, and the resulting reducing saccharide was colorimetrically determined using the Somogyi-Nelson method.

Zero point five (0.5) milliliters of a soluble starch solution and 0.4 mL of 0.1 mol/L acetic acid/sodium acetate buffer (pH 6.0) (containing 0.05 mol/L CaCl₂) were weighed into a 50 mL Nessler tube, and after shaking well and leaving at 40±0.5°C for 10 to 15 minutes, 0.1 mL of the sample solution was added and the mixture was immediately shaken. This solution was left at 40±0.5°C for exactly 15 minutes, then 1 mL of an alkaline copper test solution was added, the mixture was shaken, and stoppered, heated in a boiling water bath for exactly 20 minutes, and cooled immediately. After cooling, 1 mL of a Nelson's solution was added, and the mixture was shaken well until the red precipitate of cuprous oxide was completely dissolved, then, the mixture was left at room temperature for 20 minutes, and 22 mL of water was added, and the mixture was shaken well. The absorbance of this liquid at a wavelength of 520 nm was measured using water as a control. When reducing saccharide equivalent to 1 µmol of glucose was generated per minute, it was defined as one unit.

### [Glucose oxidase activity measurement method: Carbohydrate oxidase activity measurement method]

An appropriate amount of an enzyme was weighed, and a cooled potassium phosphate/sodium hydroxide buffer (0.1 mol/L) of pH 7.0 was added to dissolve or uniformly disperse the enzyme to make 50 mL, which was used as a sample solution. Two point five zero (2.50) grams of D(+)-glucose was weighed and dissolved with water added to make 25 mL, which was used as a substrate solution. Zero point five (0.5) milliliters of the substrate solution, 2 mL of potassium phosphate/sodium hydroxide buffer (0.1 mol/L, pH 7.0, containing phenol), 0.5 mL of a peroxidase test solution (25 units/mL) and 0.1 mL of a 4-aminoantipyrine solution (1→250) were placed in a quartz cell and heated at 37°C for 10 minutes. Zero point one (0.1) milliliters of a sample solution was added to this solution, mixed well, and heated at 37°C to prepare a test solution. Separately, a comparison solution was prepared in the same manner as in the preparation of the test solution using a pH 7.0 potassium phosphate/sodium hydroxide buffer (0.1 mol/L) or water instead of the sample solution. For the test solution and the comparison solution, the absorbance at a wavelength of 500 nm was measured 2 minutes and 5 minutes after addition of the sample solution. The amount of oxidized glucose was determined from the molar extinction coefficient of the generated quinoneimine dye. Under these conditions, the amount of the enzyme required to oxidize 1 µmol of glucose per minute was defined as 1 unit.

### 3. Experimental Example

### <Experimental Example 1>

In Experimental Example 1, a saccharide reduction effect by a maltotriose-generating enzyme was investigated.

### (1) Experimental method

Twelve point five (12.5) grams of oat flour (Slow Food Kitchen PTEMIUM OAT FLOUR) was weighed into a beaker, and water was added so that the liquid volume was 100 mL. Zero point five (0.5) percent (w/w oat flour) of α-amylase and 12 U of a maltotriose-generating enzyme per 1g of oat flour were added thereto, the beaker was placed in a 60°C water bath, and after the temperature of the solution reached 60°C, the solution was allowed to stand still for 2 hours. Thereafter, the beaker was placed in a boiling water bath and allowed to stand still for 10 minutes to inactivate the enzyme and prepare an oat drink. A control in which the maltotriose-generating enzyme was replaced with 0.3% (w/w oat flour) of β-amylase was obtained.

The prepared oat drink was measured into a 1.5 mL sample tube, 190 µL of 1 mol/L trichloroacetic acid was added thereto, and the tube was allowed to stand still at room temperature for 10 minutes. After centrifugation (10,000 rpm, 10 min), 1 mL of the supernatant was recovered into a sample tube. The operation of adding 1 mL of diethyl ether (FUJIFILM reagent special grade), mixing, and recovering 1 mL of the aqueous layer by centrifugation was repeated twice.

An equal amount of ethanol (99.5) (FUJIFILM reagent special grade) was added to the recovered water tank to prepare a sample for analysis.

The analytical sample was subjected to HPLC using MCl GEL CK04S column (manufactured by Mitsubishi Chemical Corporation) to analyze the generated saccharide. For comparison, the saccharide content of commercially available oat milk was also analyzed.

### (2) Results

The results are shown in Table 1.

**[Table 1]**

| | Enzyme addition amount (per oat flour) | | | Generated saccharide amount mg/mL | | | | Trisaccharides/ Saccharides ratio |
|---|---|---|---|---|---|---|---|---|
| | α-amylase | β-amylase | Maltotriose-generating enzyme | Monosaccharides | Disaccharides | Saccharides (monosaccharides+disaccharides) | Trisaccharides | |
| Example 1 | 0.5% | - | 6 U/g | 4.7 | 12.8 | 17.6 | 18.6 | 1.1 |
| Example 2 | 0.5% | - | 12 U/g | 4.4 | 13.8 | 18.2 | 22.5 | 1.2 |
| Comparative Example 1 | 0.5% | 0.3% | - | 1.7 | 35.9 | 37.6 | 14.3 | 0.4 |
| Comparative Example 2 | Commercially available product A | | | 0.4 | 32.2 | 32.6 | 8.8 | 0.3 |
| Comparative Example 3 | Commercially available product B | | | 0.9 | 46.6 | 47.5 | 9.1 | 0.2 |
| Comparative Example 4 | Commercially available product C | | | 30.3 | 21.6 | 52.0 | 6.0 | 0.1 |
| Comparative Example 5 | Commercially available product D | | | 2.6 | 25.3 | 27.9 | 11.1 | 0.4 |
| Comparative Example 6 | Commercially available product E | | | 34.4 | 2.6 | 37.0 | 0.0 | 0.0 |

### (3) Discussion

As shown in Table 1, the saccharide content of both the β-amylase-treated oat drink and commercially available oat milk was 27 mg/mL or more. On the other hand, it was confirmed that the amount of saccharides could be reduced to 20 mg/mL or less by using a maltotriose-generating enzyme.

### <Experimental Example 2>

In Experimental Example 2, a saccharide reducing effect by a carbohydrate oxidase was confirmed.

### (1) Experimental method

According to the method of Example 1, an oat drink using a maltotriose-generating enzyme was prepared, and 200 mL was measured and placed in a 500 mL Erlenmeyer flask. A carbohydrate oxidase derived from Acremonium chrysogenum was added thereto so that the glucose oxidase activity was 600 units, the Erlenmeyer flask was placed in a 40°C water bath, and these were stirred to react for 2 hours while supplying adequate air with a 2 mm diameter tube. After the enzyme reaction, the oat drink was placed in a boiling water bath and allowed to stand still for 10 minutes to inactivate the enzyme. The inactivated oat drink was subjected to routine treatment, and the generated saccharide was analyzed by HPLC using ICSep ICE-ION-300 column (Transgenomic Inc., New Haven, CT, USA).

### (2) Results

The results are shown in Table 2.

**[Table 2]**

| | Enzyme addition amount (per oat flour) | | | Generated saccharide amount mg/mL | | |
|---|---|---|---|---|---|---|
| | α-amylase | Maltotriose-generating enzyme | Carbohydrate oxidase | Monosaccharides | Disaccharides | Saccharides (monosaccharides+disaccharides) |
| Example 3 | 0.5% | 12 U/g | - | 3.4 | 150 | 18.4 |
| Example 4 | 0.5% | 12 U/g | 24 U/g | 3.2 | 128 | 16.0 |

### (3) Discussion

As shown in Table 2, it was confirmed that the saccharide content could be further reduced by further causing a carbohydrate oxidase to act on the maltotriose-generating enzyme-treated oat drink.

### <Experimental Example 3>

In Experimental Example 3, the effects were confirmed when the α-amylase acting step was performed before the maltotriose-generating enzyme acting step and further when various enzyme acting conditions were changed.

### (1) Experimental method

Twelve point five (12.5) grams of oat flour (Slow Food Kitchen PTEMIUM OAT FLOUR) was weighed into a beaker, and water was added to make the liquid volume 100 mL. Zero point five (0.5)% (w/w oat flour) of α-amylase was added thereto and placed in a 60°C water bath, and after the temperature of the solution reached 60°C, it was allowed to stand still for 2 hours. Thereafter, the beaker was placed in a boiling water bath and allowed to stand still for 10 minutes to deactivate the enzyme and prepare an oat drink raw material. To the oat drink raw material, 1.2 to 120 U of a maltotriose-generating enzyme per 1 g of oat flour was added, the beaker was placed in a 40°C or 50°C water bath, and after the temperature of the solution reached 60°C, it was allowed to stand still for 1 to 3 hours. Thereafter, the beaker was placed in a boiling water bath and allowed to stand still for 10 minutes to inactivate the enzyme and prepare an oat drink.

The prepared oat drink was measured into a 1.5 mL sample tube, 190 µL of 1 mol/L trichloroacetic acid was added thereto, and the tube was allowed to stand still at room temperature for 10 minutes. After centrifugation (10,000 rpm, 10 min), 1 mL of the supernatant was recovered into a sample tube. The operation of adding 1 mL of diethyl ether (FUJIFILM reagent special grade), mixing, and recovering 1 mL of the aqueous layer by centrifugation was repeated twice.

An equal amount of ethanol (99.5) (FUJIFILM reagent special grade) was added to the recovered water tank to prepare a sample for analysis.

The analytical sample was subjected to HPLC using MCl GEL CK04S column (manufactured by Mitsubishi Chemical Corporation) to analyze the generated saccharide. For comparison, the saccharide content of commercially available oat milk was also analyzed.

### (2) Results

The results are shown in Tables 3 and 4.

**[Table 3]**

| Treated at 50°C | Enzyme addition amount (per oat flour) | | Maltotriose-generating enzyme treatment time | Generated saccharide amount mg/mL | | | | Trisaccharides/ Saccharides ratio |
|---|---|---|---|---|---|---|---|---|
| | α-amylase | Maltotriose-generating enzyme | | Monosaccharides | Disaccharides | Saccharides (monosaccharides+disaccharides) | Trisaccharides | |
| Example 5 | 0.5% | 1.2 U/g | 1 hr | 1.1 | 4.5 | 5.6 | 10.0 | 1.8 |
| Example 6 | 0.5% | 1.2 U/g | 2 hr | 1.2 | 5.0 | 6.2 | 13.3 | 2.2 |
| Example 7 | 0.5% | 1.2 U/g | 3 hr | 1.2 | 5.3 | 6.4 | 15.5 | 2.4 |
| Example 8 | 0.5% | 12U/g | 1 hr | 1.5 | 7.1 | 8.5 | 24.2 | 2.8 |
| Example 9 | 0.5% | 12 U/g | 2 hr | 1.8 | 8.2 | 10.0 | 28.1 | 2.8 |
| Example 10 | 0.5% | 12 U/g | 3 hr | 2.0 | 8.6 | 10.6 | 29.2 | 2.7 |
| Example 11 | 0.5% | 120 U/g | 1 hr | 4.3 | 10.9 | 15.3 | 32.6 | 2.1 |
| Example 12 | 0.5% | 120 U/g | 2 hr | 3.9 | 10.2 | 14.1 | 28.6 | 2.0 |
| Example 13 | 0.5% | 120 U/g | 3 hr | 4.6 | 12.2 | 16.8 | 32.4 | 1.9 |
| Comparative Example 7 | Commercially available product A | | | 1.7 | 31.4 | 33.1 | 8.9 | 0.3 |

**[Table 4]**

| Treated at 40°C | Enzyme addition amount (per oat flour) | | Maltotriose-generating enzyme treSment time | Generated saccharide amount mg/mL | | | | Trisaccharides/ Saccharides ratio |
|---|---|---|---|---|---|---|---|---|
| | α-amylase | Maltotriose-generating enzyme | | Monosaccharides | Disaccharides | Saccharides(monosaccharides+disaccharides) | Trisaccharides | |
| Example 14 | 0.5% | 1.2 U/g | 1 hr | 1.6 | 4.6 | 6.2 | 9.1 | 1.5 |
| Example 15 | 0.5% | 1.2 U/g | 2 hr | 1.6 | 4.9 | 6.6 | 11.4 | 1.7 |
| Example 16 | 0.5% | 1.2 U/g | 3 hr | 1.6 | 5.1 | 6.8 | 13.3 | 2.0 |
| Example 17 | 0.5% | 12 U/g | 1 hr | 1.8 | 6.6 | 8.4 | 22.2 | 2.6 |
| Example 18 | 0.5% | 12 U/g | 2 hr | 2.3 | 7.6 | 9.9 | 26.8 | 2.7 |
| Example 19 | 0.5% | 12 U/g | 3 hr | 2.5 | 8.0 | 10.6 | 28.9 | 2.7 |
| Example 20 | 0.5% | 120 U/g | 1 hr | 4.5 | 10.0 | 14.5 | 33.4 | 2.3 |
| Example 21 | 0.5% | 120 U/g | 2 hr | 4.8 | 10.4 | 15.2 | 34.6 | 2.3 |
| Example 22 | 0.5% | 120 U/g | 3 hr | 4.8 | 10.6 | 15.4 | 34.6 | 2.2 |
| Comparative Example 7 | Commercially available product A | | | 1.7 | 31.4 | 33.1 | 8.9 | 0.3 |

### (3) Discussion

As shown in Tables 3 and 4, even when maltotriose-generating enzyme treatment was performed after α-amylase treatment, the saccharide reduction effect was confirmed. Furthermore, the saccharide reduction effect was confirmed even when the amount of the maltotriose-generating enzyme added, the treatment temperature, and the treatment time were changed.

## Claims

1. A method for producing a plant-base drink or food, which comprises a step of causing a maltotriose-generating enzyme to act on a plant-base raw material.

2. The method for producing a plant-base food or drink according to claim 1, wherein the plant-base raw material is a raw material treated with α-amylase.

3. The method for producing a plant-base food or drink according to claim 1, further comprising a step of causing a carbohydrate oxidase to act on a plant-base raw material.

4. The method for producing a plant-base food or drink according to claim 1, wherein the plant-base food or drink is oat milk.

5. An enzymatic agent for saccharide reduction comprising a maltotriose-generating enzyme.

6. The enzymatic agent for saccharide reduction according to claim 5, further comprising a carbohydrate oxidase.

7. A method for reducing saccharides in plant-base drinks or foods, which comprises a step of causing a maltotriose-generating enzyme to act on a plant-base raw material.
